**Europäisches Patentamt**

**European Patent Office**

**Office Européen des brevets**

(19)

(11) Veröffentlichungsnummer: **0 281 112 B1**

# EUROPÄISCHE PATENTSCHRIFT

(12)

(45) Veröffentlichungstag der Patentschrift:
02.01.91 Patentblatt 91/01

(51) Int. Cl.$^5$: **G01N 33/48, G01N 33/537,**
**G01N 33/76, G01N 31/00,**
**G01N 33/543**

(21) Anmeldenummer: **88103194.2**

(22) Anmeldetag: **02.03.88**

(54) **Vorrichtung zur Durchführung einer heterogenen Reaktion.**

(30) Priorität: **02.03.87 DE 3706718**

(43) Veröffentlichungstag der Anmeldung:
**07.09.88 Patentblatt 88/36**

(45) Bekanntmachung des Hinweises auf die
Patenterteilung:
**02.01.91 Patentblatt 91/01**

(84) Benannte Vertragsstaaten:
**AT BE CH DE ES FR GB GR IT LI LU NL SE**

(56) Entgegenhaltungen:
**EP-A- 0 049 898**
**EP-A- 0 230 618**
**DE-A- 3 430 905**

(73) Patentinhaber: **BOEHRINGER MANNHEIM
GMBH
Patentabteilung, Abt. E Sandhofer Strasse
112-132 Postfach 31 01 20
D-6800 Mannheim 31 Waldhof (DE)**

(72) Erfinder: **Klose, Sigmar, Dr.phil.
Breitenloh 7
D-8131 Berg 2 (DE)**
Erfinder: **Erler, Klaus, Dr.rer.nat.
Lindenberg 8
D-8134 Pöcking (DE)**
Erfinder: **Uhl, Wolfgang, Dr.-Ing.
Westendstr. 15
D-8120 Weilheim (DE)**
Erfinder: **Baier, Manfred, Dr.rer.nat.
Tiefentalweg 10
D-8124 Seeshaupt (DE)**

(74) Vertreter: **Huber, Bernhard, Dipl.-Chem. et al
Patentanwälte H. Weickmann, Dr. K. Fincke
F.A. Weickmann, B. Huber Dr. H. Liska, Dr. J.
Prechtel Möhlstrasse 22 Postfach 860 820
D-8000 München 86 (DE)**

## Beschreibung

Die Erfindung betrifft eine Vorrichtung zur Durchführung einer heterogenen Reaktion zur Bestimmung einer Komponente einer Probenflüssigkeit auf einem in einer Meßkammer (Me) befindlichen ersten Kapillaraktiven Träger (T1), an dem mindestens ein nicht lösliches Reagenz haftet.

Die Erfindung betrifft auch ein Statisches Kapillartstrom-Einwegventil für die Owen genannte Vorrichtung

Aufgabe der Erfindung ist es, eine Vorrichtung dieser Art anzugeben, mittels der allein unter Ausnutzung der Schwerkraft - allenfalls unter Drehung der Vorrichtung in unterschiedlichen Lagen - zuverlässig eine heterogene Reaktion zum Nachweis einer Komponente einer Probenflüssigkeit (Analyt) durchgeführt werden kann.

Heterogene Reaktionen zum Nachweis von Analyten, Reaktionen zwischen einem Analyten, mindestens einem löslichen und mindestens einem unlöslichen Reagenz, wobei eine oder mehrere Reaktionen ablaufen, können im allgemeinen nicht in einem Schritt durchgeführt werden. Abhängig vom Reaktionstyp muß beispielsweise überschüssiges Probenmaterial oder Reagenz entfernt werden (Waschschritt), Hilfsreagenz zugegeben werden und/oder nach Ablauf einer Reaktion (Inkubationsschritt) ein weiteres Reagenz, beispielsweise ein Indikatorreagenz, zugegeben werden.

Diese Schritte sind üblicherweise mit Tätigkeiten, wie Pipettieren, Dosieren und Homogenisieren verbunden, welche nur durch geübtes Laborpersonal zuverlässig durchführbar sind. Mit der erfindungsgemäßen Vorrichtung sollen derartige Tätigkeiten bei Analysen vermieden werden und daher auch für ungeübte Anwender Analysen unter Verwendung von heterogenen Reaktionen einfach und mit hinreichender Genauigkeit möglich sein. Auch bei nicht ganz sachgemäßer Handhabung soll die Analyse von Vorrichtung zu Vorrichtung in gleicher Weise ablaufen und somit zu gleichen Meßergebnissen führen, wobei das Meßergebnis optisch angezeigt oder in anderer noch zu erläuternder Weise ermittelt werden kann.

Eine erfindungsgemäße Vorrichtung soll insbesondere als Wegwerfvorrichtung zur Ausführung von Tests (insbesondere Schwangerschaftstests) ausgebildet werden können. Sie soll als Massenartikel hergestellt werden können und keine individuellen Eingriffe erfordern.

Die grundsätzliche Lösung dieser Aufgabe ist vorliegende Vorrichtung die dadurch gekennzeichnet, daß sich mindestens ein lösliches Reagenz auf einem mit dem Kapillaraktiven Träger (T1) in der Meßkammer (Me) in kappillarem Kontakt stehenden zweiten kapillaraktiven Träger (T2) in einer ersten Vorkammer (V1) befindet, die über eine erste Kapillare (K1) mit einer von außen durch eine Füllöffnung (F1) zu füllenden ersten Aufgabekammer (A1) verbunden ist, und daß die Meßkammer (Me) überdies durch eine Kapillaranordnung (KA), die nur von einer unter einer vorgegebenen Schwerkraft stehenden Flüssigkeit in der Meßkammer (Me) durchströmt werden kann, mit einer Abfallkammer (Abf) verbunden ist Das statische Kappilarstrom- Einwegventil ist gekennzeichnet durch eine Kammer (2), in der eine Zuströmkapillare (4) und eine Abström-kappilare (6) münden, wobei die Wandflächen (8) der Zuströmkapillare (4) mit den an sie angrenzenden Wandflächen (10, 12, 14, 16) der kammer (2) Winkel (α) einschließen, die in ihrem Mittelwert gröber sind als in ihrem Mittelwert diejenigen Winkel (ß), die die Wandflächen (18) der Abströmkapillare (6) mit den an sie angrenzenden Wandflächen (20, 22, 24) der kammer (2) einschließen.

Die Reaktionspartner im Sinne der vorliegenden Erfindung sind der Analyt, mindestens ein lösliches Reagenz und mindestens ein unlösliches Reagenz. Derartige Reagentien sind dem Fachmann geläufig und bedürfen keiner ausführlichen Erläuterung.

Besonders geeignet ist die Vorrichtung zur Durchführung von enzymatischen und/oder immunologischen Bestimmungsreaktionen, insbesondere unter Verwendung von Enzymen, Substraten, Antikörpern, Antigenen, Haptenen, Farbbildungsreagentien und Hilfsreagentien, wie Puffern oder Tensiden (z. B. zur Ablösung des Analyten von Serumbestandteilen).

In einer besonders bevorzugten Ausführungsform wird die Vorrichtung zur Durchführung von immunologischen Bestimmungsverfahren, wie kompetitive, einfache Sandwich-oder DASP-Sandwich-Tests verwendet. Derartige Verfahren sind beispielsweise in Laboratorytechniques in Biochem. and Molec. Biol., Ed. R. H. Burdon, P.H. v. Knippenberg, Elsevier, Amsterdam, Vol. 15 (1985), S. 9-37 und Lig. Rev. 3/3 (1981) S. 6-13 beschrieben. Bei derartigen Bestimmungsverfahren wird der Analyt direkt oder indirekt an das unlösliche Reagenz gebunden. Gleichzeitig oder in weiteren Schritten kann dann über eine Nachweisreaktion (z. B. Farbbildung) festgestellt werden, wieviel Analyt gebunden wurde.

Die Unteransprüche befassen sich mit zweckmäßigen Ausgestaltungen der Lehre des Anspruchs 1.

Die funktionelle Bedeutung der Merkmale der Ansprüche wird im Zusammenhang mit Ausführungsbeispielen unter Hinweis auf die beigefügten Zeichnungen erläutert.

Dabei hat Anspruch 19 mit den zu ihm gehörigen Unteransprüchen eine selbständige Bedeutung.

Fig. 1 zeigt in Aufsicht schematisch eine erste Ausführungsform der Vorrichtung.

Fig. 2 zeigt in Aufsicht schematisch eine zweite Ausführungsform der Vorrichtung.

Fig. 3 zeigt in Aufsicht schematisch eine dritte Ausführungsform der Vorrichtung.

Fig. 4 zeigt im Längsschnitt ein statisches Kapillarstrom-Einwegventil in Blickrichtung IV-IV der Fig. 5.

Fig. 5 zeigt eine Aufsicht auf das Einwegventil in der Blickrichtung V-V der Fig. 4.

Merkmale, die die Vorrichtungen gemein haben, werden nur einmal beschrieben.

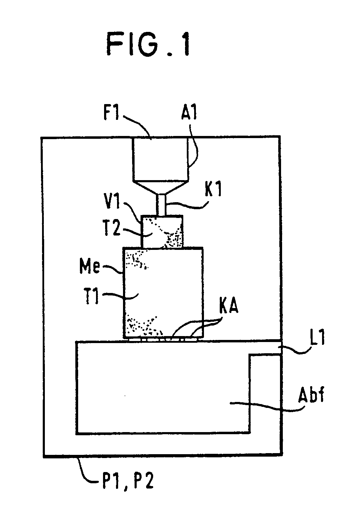

Die Vorrichtungen nach den Fig. 1 bis 3 dienen zur Durchführung einer heterogenen Reaktion zur Bestimmung einer Komponente einer Probeflüssigkeit (Analyt) auf einem in einer Meßkammer Me befindlichen ersten kapillaraktiven Träger T1, an dem mindestens ein nicht lösliches Reagenz haftet. Mindestens ein lösliches Reagenz befindet sich auf einem mit dem kapillaraktiven Träger T1 in der Meßkammer Me in kapillarem Kontakt stehenden zweiten kapillaraktiven Träger T2 in einer ersten Vorkammer V1. "Kapillarkontakt" bedeutet, daß die kapillaraktiven Träger T1 und T2 in kapillarem Kontakt stehen, so daß der kapillaraktive Träger T1 in seine Kapillaren Flüssigkeit aus den Kapillaren des kapillaraktiven Trägers T2 aufnehmen kann. Die Vorkammer V1 ist über eine erste Kapillare K1 mit einer von außen durch eine Füllöffnung F1 zu füllende erste Aufgabekammer A1 - die bei der Ausführungsform nach Fig. 1 die einzige Aufgabekammer ist - verbunden. Die Meßkammer Me ist überdies durch eine Kapillaranordnung KA, die nur von einer unter einer vorgegebenen Schwerkraft stehenden Flüssigkeit von der Richtung der Meßkammer Me aus durchströmt werden kann, mit einer Abfallkammer Abf verbunden. Dabei bildet die Kapillaranordnung KA einen Einlaß der Abfallkammer Abf.

Die Abfallkammer Abf ist mit einer Entlüftungsöffnung L1 versehen.

Die Kapillare K1 hat - was auch für die noch im folgenden zu beschreibenden Kapillare gilt - einen hydraulischen Durchmesser von 0,05 mm bis 2 mm, vorzugsweise einen hydrodynamischen Durchmesser von 0,2 mm bis 0,6 mm. (Der "hydraulische Durchmesser" ist definiert als das Vierfache des Quotienten von Querschnittsfläche und Umfang der Kapillare, wodurch auch - hier geeignete - Kapillaren mit nicht kreisförmigem Querschnitt beschrieben werden).

Als kapillaraktive Träger T1, T2 eignen sich - was auch für die noch im folgenden zu beschreibenden kapillaraktiven Träger gilt - Papier, Vlies, Gewebe, Gewirk oder poröses Membranmaterial.

Die kapillaraktiven Träger lassen sich durch ihre Saugkapazität und durch ihre Saugkraft charakterisieren. Als Maß für ihre Saugkraft läßt sich die Saughöhe verwenden, mit der sie in einer vorgegebenen Zeit (300 s) eine Flüssigkeit ansaugen. Die Saughöhe ist dabei die Höhe der Front der angesaugten Flüssigkeit über dem Spiegel der freien Flüssigkeit, in die der kapillaraktive Träger getaucht wird. Weiterhin kann die Anfangsgeschwindigkeit des Ansaugens von Bedeutung sein, etwa die Zeit für das Erreichen einer Saughöhe von 30 mm. In Einzelfällen kann auch die Luftdurchlässigkeit von Bedeutung sein.

Als Besonders geeignet haben sich kapillaraktive Träger mit folgenden Eigenschaften erwiesen :

|  | Bereich | | bevorzugt | |
| --- | --- | --- | --- | --- |
| Saugkapazität: | 400 - 1200 ml/m² | | 700 - 1100 ml/m² | |
| Saughöhe (in 300 s): | 40 - 150 mm | | 80 - 120 mm | |
| Anfangsgeschwindig-keit des Ansaugens | 4 - 40 s/30 mm | | 4 - 10 s/30 mm | |
| Luftdurchlässigkeit: | 2 - 15 l/min m² | | | |

Derartige kapillaraktive Träger sind beispielsweise in der DE 35 43 749 beschrieben. Die dort angegebene Perjodat-Aktivierung ist für die hier geeigneten Träger nicht erforderlich.

Die Kammern A1, V1, Me und Abf, die Kapillare K1 und die Kapillaranordnung KA sind - was auch für die im folgenden zu beschreibenden Kammern, Kapillaren, Kapillaranordnungen und Kapillarstrom-Einwegventile gilt - in einer ebenen Fläche einer ersten Platte P1 eingearbeitet, die von einer ebenen Fläche einer zweiten Platte P2 abgedeckt ist. Wenigstens eine der Platten P1, P2 ist dabei wenigstens im Bereich der Meßkammer Me durchsichtig, damit man die erforderliche Messung oder Beobachtung der heterogenen Reaktion ausführen kann.

Als Material für die Platten P1 und P2 hat sich Glas oder Kunststoff als besonders geeignet erwiesen, als Kunststoff besonders Polyacrylat oder Polystyrol.

Bei Anwendung der Vorrichtung nach Fig. 1 befindet sich auf dem Träger T2 ein lösliches Reagenz und auf dem Träger T1 ein unlösliches Reagenz. Zur Herbeiführung der heterogenen Reaktion wird erst eine vorgegebene Menge einer Probeflüssigkeit in die Aufgabekammer A1 gegeben. Diese Flüssigkeit löst Reagenz vom Träger T2 und strömt mit diesem zum Träger T1. Dann wird gegebenenfalls nach einer Inkubationszeit eine vorgegebene Menge eines Diluens (einer Wasch- und Elutionslösung) in die Aufgabekammer A1 gegeben, die den Träger T2 vom Reagenz im wesentlichen befreit und das Reagenz auf den Träger T1 überführt. Überschüssige Flüssigkeit gelangt dabei durch die Kapillaranordnung KA in die Abfallkammer Abf.

Die Abfallkammer enthält entweder unbelegtes Vlies oder Vlies, welches mit Reagentien imprägniert ist, die dort gegebenenfalls auch ablaufende - und unerwünschte -Nachweisreaktionen (wie Farbbildung oder Lumineszenz) unterdrücken.

Die Kapillare K1 sorgt dafür, daß eine gleichmäßige, allmähliche Verteilung der jeweiligen Flüssigkeit, die ihr durch die Aufgabekammer A1 zuströmt, auf den Träger T2 erfolgt.

Die Kapillaranordnung KA ist im wesentlichen ein eindimensionales Gitter, das aufgrund der Oberflächenspannung der von oben zu den Gitteröffnungen gelangenden Flüssigkeit die Flüssigkeit zunächst nicht durchläßt, sondern erst dann, wenn die Flüssigkeit auf die Gitteröffnungen mit einem vorgegebenen, durch die Schwerkraft bedingten Druck wirkt. Die Kapillaranordnung KA hat zur Folge, daß sich der Träger T1 mit einer vorgegebenen Menge des Reaktionsprodukts oder Gemischs aus Analyt und löslichem Reagenz füllt.

Anschließend wird eine vorgegebene Menge eines weiteren Reagenzes in die Aufgabekammer A1 gegeben, das mit den auf dem Träger T1 befindlichen Reagentien reagiert.

Die Reaktion kann etwa durch Färbung des Trägers T1 beobachtet werden. Es kann aber auch eine Messung mittels Reflektometrie erfolgen oder durch Messung von Fluoreszenz oder Lumineszenz, wenn ein Reagenz oder das Diluens einen fluorogenen oder luminogenen Bestandteil enthält.

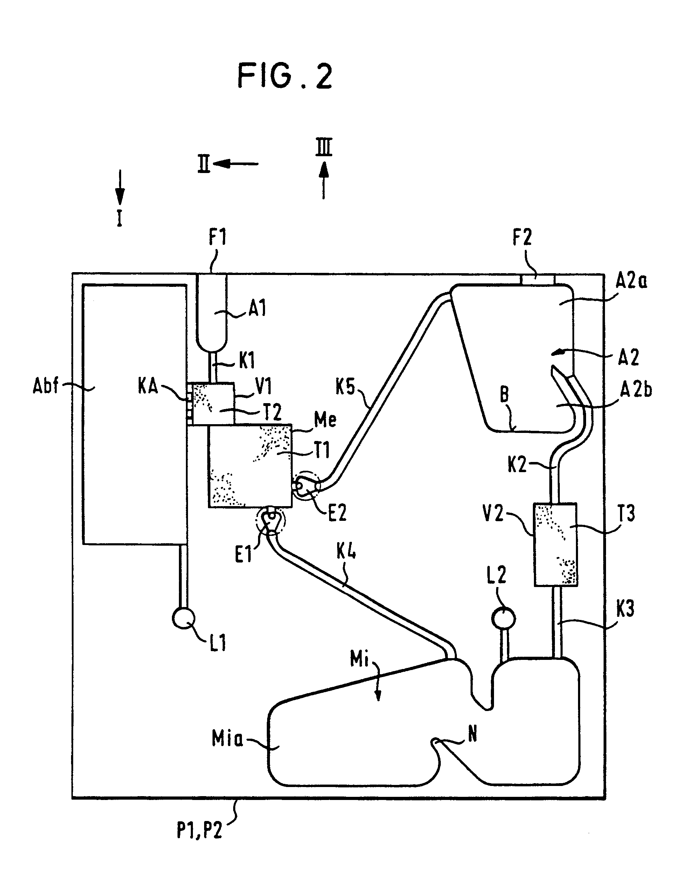

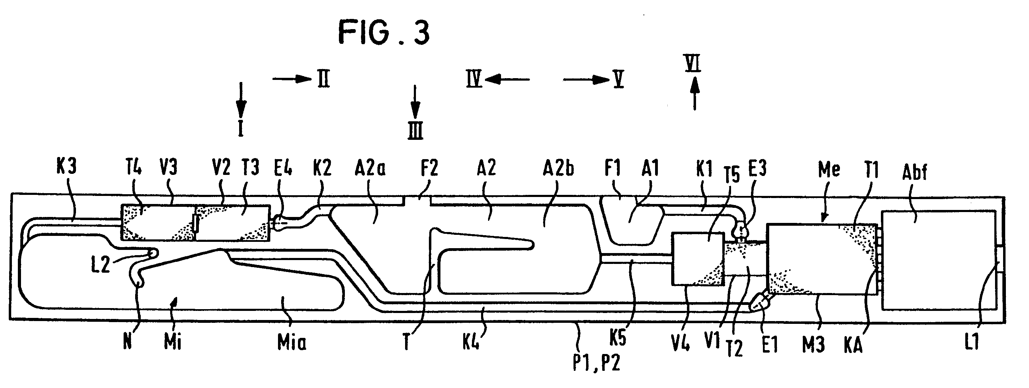

Bei den Ausführungsformen nach den Fig. 2 und 3 ist eine zweite, von außen durch eine Füllöffnung F2 zu füllende Aufgabekammer A2 über eine zweite Kapillare K2 mit einer zweiten Vorkammer V2 verbunden, in der sich wenigstens ein weiteres Reagenz auf einem dritten kapillaraktiven Träger T3 befindet. Über eine dritte Kapillare K3 ist die zweite Vorkammer V2 mit einer leeren Mischkammer Mi verbunden, die ihrerseits über eine vierte Kapillare K4 mit der Meßkammer M3 verbunden ist.

In die Mischkammer Mi ragt eine Nase N, über die aus der dritten Kapillare K3 in die Mischkammer Mi eingetretene Flüssigkeit beim Kippen der Mischkammer Mi in einen Raumteil Mia der Mischkammer Mi fließt, von dem die vierte Kapillare K4 abgeführt ist. Die zweite Aufgabekammer A2 ist überdies über eine fünfte Kapillare K5 mit der Meßkammer Me verbunden.

Dabei geht bei der Ausführungsform nach Fig. 2 die zweite Kapillare K2 von einem Bereich der zweiten Aufgabekammer A2 aus, der etwa mittig zwischen deren Füllöffnungen F2 und deren der Füllöffnung F2 gegenüberliegenden Boden B liegt, so daß die Aufgabekammer A2 in der in Fig. 2 dargestellten Lage der Vorrichtung in einen oberen Bereich A2a und einen unteren Bereich A2b aufgeteilt wird. Die fünfte Kapillare K5 geht von einer Stelle in dem oberen Bereich A2a der zweiten Aufgabekammer A2 aus, die etwa in Höhe der Füllöffnung F2 liegt.

Die Kapillaren K4 und K5, die zu der den kapillaren Träger T1 enthaltenden Meßkammer Me führen, sind über jeweils ein nur in Richtung zu dieser Meßkammer Me zu durchströmendes statisches Kapillarstrom-Einwegventil E1 bzw. E2 mit der Meßkammer Me verbunden, um zu verhindern, daß die Kapillaren K4 und K5 Flüssigkeit, die durch sie auf den Träger T1 gelangt, wieder aufsaugen. Besonders wichtig ist, daß Flüssigkeit, die durch die Kapillare K4 zum Träger T1 in der Meßkammer Me gelangt, nicht durch die Kapillare K5 aufgesaugt wird und daß Flüssigkeit, die durch die Kapillare K5 zum Träger T1 in der Meßkammer Me gelangt, nicht durch die Kapillare K4 aufgesaugt wird.

Bei dieser Ausführungsform ist auch die Mischkammer Mi mit einer Entlüftungsöffnung L2 versehen.

Bei Anwendung der Ausführungsform nach Fig. 2 werden in der in Fig. 2 dargestellten Lage (Pfeil I weist nach unten) die Aufgabekammer A1 mit Probeflüssigkeit und die Aufgabekammer A2 mit Diluens gefüllt. Die Aufgabekammer A1 entleert sich durch die Kapillare K1 über den Träger T2, auf dem sich das lösliche Reagenz befindet, zum Träger T1.

Das Diluens im oberen Bereich A2a der Aufgabekammer A2 eluiert den Träger T3, auf dem sich ein weiteres lösliches Reagenz befindet, in die Mischkammer Mi. Das zunächst im unteren Bereich A2b der Aufgabekammer A2 verbleibende Diluens wird später über die Kapillare K5 zum Träger T1 entleert. Bei dieser Ausführungsform erfolgt das Auswaschen der Träger T1 und T2 mit dem Diluens, das über die Kapillaren K4 und K5 zuströmt und an dem Träger T1 nicht fixiertes Reagenz mitnimmt, im Gegenstrom zur Fließrichtung der Probeflüssigkeit.

Das Diluens, das mit dem löslichen Reagenz von T3 bzw. durch die Kapillare K3 in die Mischkammer Mi gelangt, fließt beim Kippen der Vorrichtung in den Zustand, in den der Pfeil II nach unten zeigt, über die Nase N in den Bereich Mia, wodurch das Diluens mit dem Indikatorreagenz homogenisiert wird.

## 1. Reaktionsbeispiel

Dieses Beispiel beschreibt einen heterogenen Immunoassay für LH (humanes luteinisierendes Hormon) nach dem DASP-Prinzip. Dieses Prinzip und die Herstellung der Reagenzien, beispielsweise der monoklonalen Antikörper und Konjugate, ist beispielsweise in der EP 0098590 und der dort zitierten Literatur zum Stand der Technik beschrieben.

## Ausgangszustand :

Als Vliese wurden verwendet :
Vlies A : 40% Linters, 60% Polyamid
Vlies B : 40% Polyamid, 30% Viskose, 20% Linters,
Vlies C : 80% Polyester, 20% Sulfitzellstoff.
In der Abfallkammer Abf befinden sich vier Vliese A 10 mm × 25 mm, 0,8 mm dick), unbelegt.
In der Vorkammer V1 befindet sich ein Vlies B (4 mm × 4 mm, 0,5 mm dick), imprägniert mit den löslichen Reagentien.

100 mM HEPES pH 7,2
1% Rinderserumalbumin (RSA)
0,9% NaCl
30 mU/Test Konjugat aus monoklonalem Antikörper (Maus, MAK1 NCACC 84 122 005) gegen LH und ß-Galactosidase
250 ng/Test monoklonaler Antikörper (Maus) gegen LH, gerichtet gegen ein anderes Epitop (MAK2 NCACC 84 122 001

Die monoklonalen Antikörper MAK1 und MAK2 sind unter den oben genannten Hinterlegungsnummern bei der European Collection of Animal Cell Cultures, GB, hinterlegt.
In der Meßkammer Me befindet sich ein Vlies C (8 mm × 8 mm, 0,6 mm dick), imprägniert mit

1% RSA
39 µg/Test polyklonaler Antikörper (Schaf) gerichtet gegen Fcγ-Teil von Maus IgG (unlösliches Reagenz).

In der zweiten Vorkammer V2 befindet sich ein Vlies B 84 mm × 8 mm, 0,5 mm dick), imprägniert mit Indikatorreagenz

70 mH HEPES pH 7,2
2 mM Mg-L-Aspartat
2 mM Borsäure
0,3% (w/v) RSA
4,0 mM Chlorphenolrot-ß-Galactosid (EP 0146866)
1,5‰ Tween 20

Die Beladung der Vliese (Imprägnierung) erfolgt wie in DE 35 43 749 beschrieben, jedoch ohne Perjodataktivierung.

## 1. Schritt :

In dem in Fig. 2 dargestellten Zustand (Pfeil I weist nach unten) werden 15 µl LH -haltiges Serum in die Aufgabekammer A1 gegeben. Das Serum eluiert das Vlies T2 und gelangt auf das Vlies T1.

## 2. Schritt :

Die Aufgabekammer A2 wird mit 450 µl 0,9% NaCl-Lösung gefüllt. Der im oberen Bereich A2a der Aufgabekammer A2 befindliche Anteil dieser Lösung strömt durch die Kapillare K2, das Vlies T3 und die Kapillare K3 nach unten in die Mischkammer Mi.

### 3. Schritt:

Nach 10 min ist die Inkubation auf dem Vlies T1 beendet und die Vorrichtung wird so gedreht, daß der Pfeil II nach unten weist. Dadurch strömt die aus der Kapillare K3 in die Mischkammer Mi gelangte Flüssigkeit über die Nase N in den Bereich Mia der Mischkammer Mi, was eine Homogenisierung der Flüssigkeit zur Folge hat.

Das Diluens aus dem unteren Bereich A2b der Aufgabekammer A2 strömt durch die Kapillare K5 über das Einwegventil E2 in die Meßkammer Me und anschließend in die Vorkammer V1, wodurch sie die Vliese T1 und T2 auswäscht. Anschließend gelangt die Flüssigkeit durch die Kapillaranordnung KA in die Abfallkammer Abf.

### 4. Schritt:

Die Vorrichtung wird so gedreht, daß der Pfeil III nach unten weist. Das Diluens mit dem Indikatorreagenz fließt dadurch aus der Mischkammer Mi über die Kapillare K4 und das Einwegventil E1 in die Meßkammer Me, so daß die Nachweisreaktion auf dem Vlies T1 herbeigeführt wird.

### 5. Schritt:

Die Nachweisreaktion auf dem Vlies T1 wird in oben beschriebener Weise beobachtet oder gemessen.

Bei der Ausführungsform der Vorrichtung nach Fig. 3 liegt die zweite Vorkammer V2 in Reihe mit einer dritten Vorkammer V3, in der sich ein Hilfsreagenz mit einem vierten kapillaraktiven Träger T4 befindet (Vlies B, 6 mm × 6 mm, 0,5 mm dick, imprägniert mit 1,5‰ Tween 20 (w/v)). Außerdem ist die zweite Aufgabekammer A2 über eine vierte Vorkammer V4, in der sich ein kapillaraktiver Träger T5 befindet, mit der ersten Vorkammer V1 verbunden. Die zweite Aufgabekammer A1 ist durch eine Trennwand T in zwei Bereiche A2a und A2b unterteilt. Der eine Bereich A2a ist über die zweite Kapillare K2 und ein Einwegventil E4 mit der zweiten Vorkammer V2 verbunden, und außerdem führt in diesen Bereich A2a die Füllöffnung F2. Der andere Bereich A2b ist über die fünfte Kapillare K5 mit der vierten Vorkammer V4 verbunden.

Die Aufgabekammer A1 dient zur Aufnahme der Probeflüssigkeit, die Aufgabekammer A2 zur Aufnahme des Diluens. Das Diluens verteilt sich auf die Bereiche A2a und A2b.

Hilfs- und Indikatorreaktionen sowie die Nachweisreaktion erfolgen auf den Trägern T5, T2 und T1.

Das Diluens strömt aus der Aufgabekammer A2 durch die Vorkammern V2 und V3 in die Mischkammer Mi. (Die Nase N in Fig. 3 ist nur als Schutz gegen Auslaufen der Flüssigkeit über das Entlüftungsloch L2 vorgesehen und dient nicht zur Homogenisierung). Die Kapillare K4, die von der Mischkammer Mi zur Meßkammer Me führt, kann gegebenenfalls durch wenigstens eine weitere Vorkammer unterbrochen sein, in der sich ein kapillaraktiver Träger befindet, beispielsweise zur Steuerung der Strömungsgeschwindigkeit und zur Beimischung von Inhibitoren zum Strömungsfrontbereich der aus der Mischkammer Mi zuströmenden Flüssigkeit.

### 2. Reaktionsbeispiel

#### Ausgangszustand:

In der Abfallkammer Abf befinden sich vier Vliese A CSP 122K (11,2 mm × 12,8 mm, 0,8 mm dick).

In der vierten Vorkammer V4 befindet sich ein Vlies B (6 mm × 6 mm, 0,5 mm dick), belegt mit 1,5‰ (w/w) Tween 20.

In der ersten Vorkammer V1 befindet sich ein Vlies B (4 mm × 4 mm, 0,5 mm dick), belegt wie im Beispiel 1.

In der Meßkammer Me befindet sich ein Vlies C (8 mm × 12 mm, 0,6 mm dick), belegt wie im Beispiel 1.

In der zweiten und dritten Vorkammer befindet sich jeweils ein Vlies B (5 mm × 10 mm, 0,5 mm dick), belegt wie das Vlies in der zweiten Vorkammer von Beispiel 1.

#### 1. Schritt:

In dem in Fig. 3 dargestellten Zustand (Pfeil I weist nach unten) wird 15 µl LH -haltiges Serum in die Aufgabekammer A1 eingegeben. Die Vorrichtung wird daraufhin so gedreht, daß der Pfeil II nach unten weist. Das Serum spült dadurch die auf dem Vlies T2 befindlichen Reagenzien auf das Vlies T1 in der Meßkammer Me. Das Vlies T1 kann die zuströmende Flüssigkeitsmenge vollständig aufnehmen. Anschließend wird die Vorrichtung in den in Fig. 3 dargestellten Zustand (Pfeil III weist nach unten) zurückgeführt.

2. Schritt :

Die Aufnahmekammer A2 wird mit 400 μl 0,9%iger NaCl-Lösung gefüllt. Diese Lösung verteilt sich auf die Bereiche A2A und A2b der Aufgabekammer A2. Zugleich erfolgt auf dem Vlies T1 die Ausbildung eines Sandwichkomplexes aus MAK2, Analyt und Konjugat und dessen Bindung an das unlösliche Reagenz über MAK2.

3. Schritt :

Die Vorrichtung wird so gedreht, daß der Pfeil IV nach unten weist. Das Diluens aus dem Bereich A2a der Aufgabekammer A2 eluiert die Vliese T3 und T4 und gelangt in die Mischkammer Mi.

4. Schritt :

Nach Ablauf einer Inkubationsreaktion auf dem Vlies T1 (10 min) wird die Vorrichtung über die in Fig. 3 dargestellte Ausgangslage hinaus gedreht, so daß der Pfeil V nach unten weist. Das Diluens aus dem Bereich A2b der Aufgabekammer A2 eluiert das Vlies T5 und fließt durch die Vliese T2 und T1 in die Abfallkammer Abf, wobei es die auf den Vliesen T2 und T1 nicht fixierten Reagenzien mitnimmt. Zugleich erfolgt eine weitgehende Homogenisierung der Flüssigkeit in der Mischkammer Mi. Falls notwendig, kann die Homogenisierung durch mehrfaches Kippen verbessert werden.

5. Schritt :

Die im 4. Schritt beschriebene Drehung wird fortgesetzt, maximal bis der Pfeil VI nach unten weist, also die Öffnungen F1 und F2 unten liegen. Es muß jedoch nicht so weit gedreht werden ; das Strömen erfolgt schon vorher, insbesondere auch schon während der Drehung. Im Ergebnis strömt allmählich das Diluens mit den Indikatorreagenzien aus der Mischkammer Mi durch die Kapillare K4 und das Einwegventil E1 in die Meßkammer Me, und es erfolgt die gewünschte Nachweisreaktion auf dem Vlies T1.

6. Schritt :

Die Reaktion auf dem Vlies T1 wird in der oben beschriebenen Weise gemessen.

Das Füllen und Kippen der Vorrichtung kann nach vorgegebenen Programmen in einem Füll- und Kippgerät erfolgen.

Sollen in den beschriebenen Kammern enthaltene, bereits feuchte Vliese erneut angeströmt werden, wird dies durch Unterlegen der Vliese mit Kunststoffnetzen erleichtert. Auch kann die Verwendung hydrophober Vliese (teflonisierter Vliese) zu diesem Zweck vorteilhaft sein oder die Einarbeitung von Entlüftungshilfen, die vorteilhaft als Gitterstrukturen ausgebildet sind, in die Kammerböden. Der Einfluß des Neigungswinkels auf die Strömungsgeschwindigkeit in den Kapillaren kann durch Bremsvliese modifiziert werden.

Das Homogenisieren der Flüssigkeit in der Mischkammer kann auch durch Ultraschall oder Gasentwicklung (beispielsweise $CO_2$) erfolgen, wobei dann die Vorrichtung hierfür nicht geschwenkt zu werden braucht.

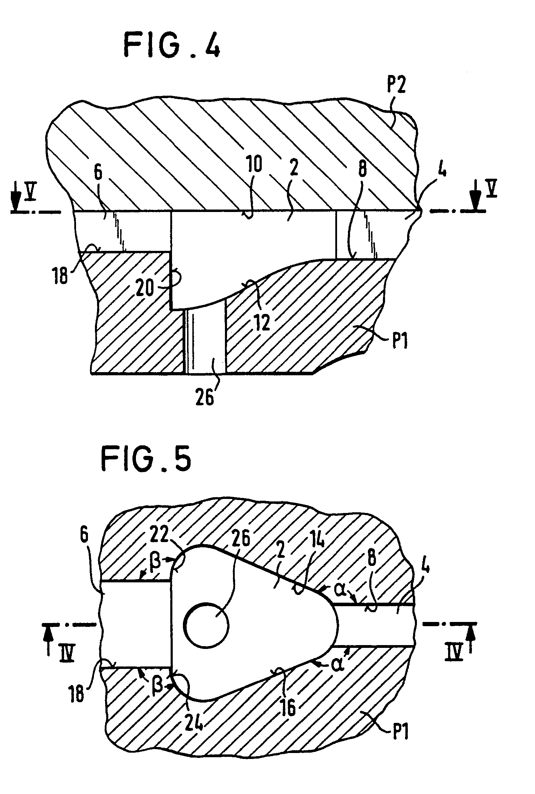

Ein bevorzugtes statistisches Kapillarstrom-Einwegventil, das als Einwegventil E1, E2, E3, E4 verwendet werden kann, weist nach Fig. 4 und 5 eine Kammer 2 auf, in der eine Zuströmkapillare 4 und eine Abströmkapillare 6 münden. Die Wandflächen 8 der Zuströmkapillare 4 schließen mit den an sie angrenzenden Wandflächen 10, 12, 14, 16 der Kammer 2 Winkel α ein, die in ihrem Mittelwert größer sind als in ihrem Mittelwert diejenigen Winkel ß, die die Wandflächen 18 der Abströmkapillare 6 mit den an sie angrenzenden Wandflächen 20, 22, 24 der Kammer 2 einschließen. Bevorzugt sind die Scheitel der Winkel α abgerundet.

In dem Ausführungsbeispiel mündet die Zustromkapillare 4 im Scheitelbereich von spitzwinklig auseinanderstrebenden Seitenflächen 14, 16 der Kammer 2 und die Abströmkapillare 6 rechtwinklig in eine dem Scheitelbereich gegenüberliegende Seitenfläche 20, 22, 24 der Kammer in Flucht mit der Zuströmkapillare 4. Die Kammer 2 weist eine Bodenfläche 12 auf, in der ein Entlüftungskanal 26 mündet.

Durch die Zuströmkapillare 4 zuströmende Flüssigkeit wird durch die Oberflächenspannung ihrer Front nicht daran gehindert, in die Kammer 2 einzutreten, was durch den Entlüftungskanal 26 noch unterstützt wird, und tritt dann auch in die Abströmkapillare 6 ein. Versucht Flüssigkeit, aus der Abströmkapillare 6 in die Kammer 2 einzutreten, so wird sie durch den abrupten Übergang der Abströmkapillare 6 zu den Seitenflächen 20, 22, 24 infolge ihrer Oberflächenspannung daran gehindert.

## Ansprüche

1. Vorrichtung zur Durchführung einer heterogenen Reaktion zur Bestimmung einer Komponente einer Probenflüssigkeit auf einem in einer Meßkammer (Me) befindlichen ersten kapillaraktiven Träger (T1), an dem mindestens ein nicht lösliches Reagenz haftet, dadurch gekennzeichnet, daß sich mindestens ein lösliches Reagenz auf einem mit dem kapillaraktiven Träger (T1) in der Meßkammer (Me) in kapillarem Kontakt stehenden zweiten kapillaraktiven Träger (T2) in einer ersten Vorkammer (V1) befindet, die über eine erste Kapillare (K1) mit einer von außen durch eine Füllöffnung (F1) zu füllenden ersten Aufgabekammer (A1) verbunden ist, und daß die Meßkammer (Me) überdies durch eine Kapillaranordnung (KA), die nur von einer unter einer vorgegebenen Schwerkraft stehenden Flüssigkeit in der Meßkammer (Me) durchströmt werden kann, mit einer Abfallkammer (Abf) verbunden ist.

2. Vorrichtung nach Anspruch 1, dadurch gekennzeichnet, daß eine zweite von außen durch eine zweite Füllöffnung (F2) zu füllende Aufgabekammer (A2) über eine zweite Kapillare (K2) mit einer zweiten Vorkammer (V2) verbunden ist, in der sich das wenigstens eine Indikatorreagenz auf einem dritten kapillaraktiven Träger (T3) befindet und die über eine dritte Kapillare (K3) mit einer leeren Mischkammer (Mi) verbunden ist, die ihrerseits über eine vierte Kapillare (K4) mit der Meßkammer (Me) verbunden ist.

3. Vorrichtung nach Anspruch 2, dadurch gekennzeichnet, daß in die Mischkammer (Mi) eine Nase (N) hineinragt, über die aus der dritten Kapillare (K3) in die Mischkammer (Mi) eingetretene Flüssigkeit beim Kippen der Mischkammer (Mi) in einen Raumteil (Mia) der Mischkammer (Mi) fließt, von dem die vierte Kapillare (K4) abgeführt ist.

4. Vorrichtung nach Anspruch 2 oder 3, dadurch gekennzeichnet, daß die zweite Aufgabekammer (A2) über eine fünfte Kapillare (K5) mit der Meßkammer (Me) verbunden ist.

5. Vorrichtung nach einem der Ansprüche 2 bis 4, dadurch gekennzeichnet, daß die zweite Kapillare (K2) von einem Bereich der zweiten Aufgabekammer (A2) ausgeht, der etwa mittig zwischen deren Füllöffnung (F2) und deren der Füllöffnung (F2) gegenüberliegenden Boden (B) liegt.

6. Vorrichtung nach einem der Ansprüche 2 bis 5, dadurch gekennzeichnet, daß die fünfte Kapillare (K5) von einem Bereich (A2a) der zweiten Aufgabekammer (A2) ausgeht, der etwa in der Höhe ihrer Füllöffnung (F2) liegt.

7. Vorrichtung nach einem der vorhergehenden Ansprüche, dadurch gekennzeichnet, daß die Kapillaranordnung (KA) einen Einlaß der Abfallkammer (Abf) bildet.

8. Vorrichtung nach einem der vorhergehenden Ansprüche, dadurch gekennzeichnet, daß wenigstens eine derjenigen Kapillaren (K1, K2, K4, K5), die zu einer einen kapillaren Träger (T2, T3, T1) enthaltenden Kammer (V1, V2, Me) führt, über ein nur in Richtung zu dieser Kammer (V1, V2, Me) zu durchströmendes statisches Kapillarstrom-Einwegventil (E1, E2, E3, E4) mit der Kammer (V1, V2, Me) verbunden ist.

9. Vorrichtung nach einem der vorhergehenden Ansprüche, dadurch gekennzeichnet, daß die Abfallkammer (Abf) und/oder die Mischkammer (Mi) mit jeweils wenigstens einer Entlüftungsöffnung (L1, L2) versehen ist.

10. Vorrichtung nach einem der vorhergehenden Ansprüche, dadurch gekennzeichnet, daß die zweite Vorkammer (V2) in Reihe mit einer dritten Vorkammer (V3) liegt, in der sich mindestens ein Hilfsreagenz auf einem vierten kapillaraktiven Träger (T4) befindet.

11. Vorrichtung nach einem der vorhergehenden Ansprüche, dadurch gekennzeichnet, daß die zweite Aufgabekammer (A2) über eine vierte Vorkammer (V4), in der sich ein kapillaraktiver Träger (T5) befindet, mit der ersten Vorkammer (V1) verbunden ist.

12. Vorrichtung nach einem der vorhergehenden Ansprüche, dadurch gekennzeichnet, daß die zweite Aufgabekammer (A2) durch eine Trennwand (T) in zwei Bereiche (A2a, A2b) unterteilt ist, von denen der eine A2a) mit der zweiten Kapillare (K2) und mit der zweiten Füllöffnung (F2) verbunden ist und von denen der andere (A2b) mit der fünften Kapillare (K5) verbunden ist.

13. Vorrichtung nach einem der vorhergehenden Ansprüche, dadurch gekennzeichnet, daß die Kapillaren (K1, K2, K3, K4, K5) einen hydraulischen Durchmesser von 0,05 bis 2 mm aufweisen.

14. Vorrichtung nach Anspruch 13, dadurch gekennzeichnet, daß die Kapillaren (K1, K2, K3, K4, K5) einen hydraulischen Durchmesser von 0,2 bis 0,6 mm aufweisen.

15. Vorrichtung nach einem der vorhergehenden Ansprüche, dadurch gekennzeichnet, daß die kapillaraktiven Träger (T1, T2, T3, T4, T5) aus Papier, Vlies, Gewebe, Gewirk oder porösem Membranmaterial bestehen.

16. Vorrichtung nach einem der vorhergehenden Ansprüche, dadurch gekennzeichnet, daß die Kammern (A1, A2, Me, Mi, Abf. V1, V2, V3, V4), die Kapillaren (K1, K2, K3, K4, K5), die Kapillaranordnung (KA) und die Kapillarstrom-Einwegventile (E1, E2, E3, E4) in einer Fläche einer ersten Platte (P1) eingearbeitet sind, die von einer zweiten Platte (P2) abgedeckt ist, wobei wenigstens eine der Platten (P1, P2) wenigstens im Bereich der Meßkammer (Me) durchsichtig ist.

17. Vorrichtung nach Anspruch 16, dadurch gekennzeichnet, daß die Platten (P1, P2) aus Glas oder Kunststoff bestehen.

18. Vorrichtung nach Anspruch 17, dadurch gekennzeichnet, daß die Platten (P1, P2) aus Polyacrylat oder Polystyrol bestehen.

19. Statisches Kapillarstrom-Einwegventil, insbesondere für eine Vorrichtung nach einem der vorhergehenden Ansprüche, gekennzeichnet durch eine Kammer (2), in der eine Zuströmkapillare (4) und eine Abströmkapillare (6) münden, wobei die Wandflächen (8) der Zuströmkapillare (4) mit den an sie angrenzenden Wandflächen (10, 12, 14, 16) der Kammer (2) Winkel ($\alpha$) einschließen, die in ihrem Mittelwert größer sind als in ihrem Mittelwert diejenigen Winkel ($\beta$), die die Wandflächen (18) der Abströmkapillare (6) mit den an sie angrenzenden Wandflächen (20, 22, 24) der Kammer (2) einschließen.

20. Ventil nach Anspruch 19, dadurch gekennzeichnet, daß die Kammer (2) spitzwinklig auseinander strebende Seitenflächen (14, 16) aufweist, in deren Scheitelbereich die Zuströmkapillare (4) mündet, und eine dem Scheitelbereich gegenüberliegende Seitenfläche (20, 22, 24), in deren Mittelbereich die Abströmkapillare (6) rechtwinklig mündet.

21. Ventil nach Anspruch 19 oder 20, dadurch gekennzeichnet, daß die Kammer (2) eine Bodenfläche (12) aufweist, in der ein Entlüftungskanal (26) mündet.

## Claims

1. Device for the carrying out of a heterogeneous reaction for the determination of a component of a sample liquid on a first capillary-active carrier (T1), present in a measurement chamber (Me), to which adheres at least one non-soluble reagent, characterised in that at least one soluble reagent is present on a second capillary-active carrier (T2) in a first pre-chamber (V1), which carrier (T2) stands in capillary contact with the capillary-active carrier (T1) in the measurement chamber (Me) which first pre-chamber (V1) isconnected via a first capillary (K1) with a first application chamber (A1) to be filled from outside through a filling opening (F1) and that the measurement chamber (Me) is also connected with a waste chamber (Abf) by a capillary arrangement (KA) through which can only flow a liquid under a predetermined gravitational force into the measurement chamber (Me).

2. Device according to claim 1, characterised in that a second application chamber (A2) to be filled from outside through a second filling opening (F2) is connected via a second capillary (K2) with a second pre-chamber (V2) in which is present at least one indicator reagent on a third capillary active carrier (T3) and which is connected via a third capillary (K3) with an empty mixing chamber (Mi) which, in turn, is connected via a fourth capillary (K4) with the measurement chamber (Me).

3. Device according to claim 2, characterised in that a nose (N) projects into the mixing chamber (Mi) over which liquid entering from the third capillary (K3) into the mixing chamber flows, upon tilting of the mixing chamber (Mi), into a chamber part (Mia) of the mixing chamber (Mi) from which leads off the fourth capillary (K4).

4. Device according to claim 2 or 3, characterised in that the second application chamber (A2) is connected via a fifth capillary (K5) with the measurement chamber (Me).

5. Device according to one of claims 2 to 4, characterised in that the second capillary (K2) starts from a region of the second application chamber (A2) which lies about in the middle between its filling opening (F2) and its bottom (B) lying opposite the filling opening (F2).

6. Device according to one of claims 2 to 5, characterised in that the fifth capillary (K5) starts from a region (A2a) of the second application chamber (A2) which lies at about the height of its filling opening (F2).

7. Device according to one of the preceding claims, characterised in that the capillary arrangement (KA) forms an inlet of the waste chamber (Abf).

8. Device according to one of the preceding claims, characterised in that at least one of those capillaries (K1, K2, K4, K5) which leads to a chamber (V1, V2, Me) containing a capillary carrier (T2, T3, T1) is connected with the chamber (V1, V2, Me) via a static capillary flow one-way valve (E1, E2, E3, E4) through which flow can only take place in the direction of this chamber (V1, V2, Me).

9. Device according to one of the preceding claims, characterised in that the waste chamber (Abf) and/or

EP 0 281 112 B1

the mixing chamber (Mi) is provided in each case with at least one ventilation opening (L1, L2).

10. Device according to one of the preceding claims, characterised in that the second pre-chamber (V2) lies in series with a third pre-chamber (V3) in which is present at least one adjuvant reagent on a fourth capillary-active carrier (T4).

11. Device according to one of the preceding claims, characterised in that the second application chamber (A2) is connected via a fourth pre-chamber (V4), in which is present a capillary-active carrier (T5), with the first pre-chamber (V1).

12. Device according to one of the preceding claims, characterised in that the second application chamber (A2) is divided by a separating wall (T) into two regions (A2a and A2b) of which (A2a) is connected with the second capillary (K2) and with the second filling opening (F2) and of which the other (A2b) is connected with the fifth capillary (K5).

13. Device according to one of the preceding claims, characterised in that the capillaries (K1, K2, K3, K4, K5) have a hydraulic diameter of 0.05 to 2 mm.

14. Device according to claim 13, characterised in that the capillaries (K1, K2, K3, K4, K5) have a hydraulic diameter of 0.2 to 0.6 mm.

15. Device according to one of the preceding claims, characterised in that the capillary-active carriers (T1, T2, T3, T4, T5) consist of paper, fleece, fabric, woven textile or porous membrane material.

16. Device according to one of the preceding claims, characterised in that the chambers (A1, A2, Me, Mi, Abf, V1, V2, V3, V4), the capillaries (K1, K2, K3, K4, K5), the capillary arrangement (KA) and the capillary flow one-way valves (E1, E2, E3, E4) are incorporated into a surface of a first plate (P1) which is covered by a second plate (P2), whereby at least one of the plates (P1, P2) is transparent at least in the region of the measurement chamber (Me).

17. Device according to claim 16, characterised in that the plates (P1, P2) consist of glass or synthetic material.

18. Device according to claim 17, characterised in that the plates (P1, P2) consist of polyacrylate or polystyrene.

19. Static capillary flow one-way valve, especially for a device according to one of the preceding claims, characterised by a chamber (2) into which opens an inflow capillary (4) and a flow-off capillary (6), whereby the wall surfaces (8) of the inflow capillary (4) form with the wall surfaces (10, 12, 14, 16) of the chamber (2) angles $\alpha$ which, on average, are greater than the average values of those angles $\beta$ formed by the wall surfaces (18) of the flow-off capillary (6) with the wall surfaces (20, 22, 24) of the chamber (2) bounding them.

20. Valve according to claim 19, characterised in that the chamber (2) has side surfaces (14, 16) at an acute angle to one another, in the apex region of which opens the inflow capillary (4), and a side surface (20, 22, 24) lying opposite the apex region, in the middle region of which side surface opens the flow-off capillary (6) at right-angles.

21. Valve according to claim 19 or 20, characterised in that the chamber (2) has a bottom surface (12) into which opens a ventilation canal (26).

**Revendications**

1. Dispositif pour la réalisation d'une réaction hétérogène en vue du détermination d'un constituant d'un échantillon liquide sur un premier support à effet capillaire (T1) placé dans une chambre de mesure (Me), auquel adhère au moins un réactif non soluble, caractérisé en ce qu'au moins un réactif soluble se trouve sur un deuxième support à effet capillaire (T2) dans une première chambre intermédiaire (V1) en contact capillaire avec le support à effet capillaire (T1) dans la chambre de mesure (Me), ladite première chambre intermédiaire étant reliée par l'intermédiaire d'un premier capillaire (K1) à une première chambre d'alimentation (A1) devant être chargée de l'extérieur à travers un orifice de remplissage (F1) et qu'en outre la chambre de mesure (Me) est reliée à une chambre à résidus (Abf) par l'intermédiaire d'un réseau de capillaires (KA) qui ne peut être traversé que par un liquide soumis à une force de pesanteur prédéterminée dans la chambre de mesure (Me).

2. Dispositif selon la revendication 1, caractérisé en ce qu'une deuxième chambre d'alimentation (A2) devant être chargée de l'extérieur à travers un deuxième orifice de remplissage (F2) est reliée, par l'intermédiaire d'un deuxième capillaire (K2), à une deuxième chambre intermédiaire (V2) dans laquelle au moins un réactif indicateur se trouve placé sur un troisième support à effet capillaire (T3) et qui est reliée à travers un troisième capillaire (K3) à une chambre de mélange vide (Mi) qui est reliée à son tour à la chambre de mesure (Me) par le biais d'un quatrième capillaire (K4).

10

3. Dispositif selon la revendication 2, caractérisé en ce qu'un bec (N) débouche dans la chambre de mélange (Mi), par lequel, lors du basculement de la chambre de mélange (Mi), le liquide introduit dans la chambre de mélange (Mi) à partir du troisième capillaire (K3) s'écoule dans un compartiment (Mia) de la chambre de mélange (Mi) d'où part le quatrième capillaire (K4).

4. Dispositif selon la revendication 2 ou 3, caractérisé en ce que la deuxième chambre d'alimentation (A2) est reliée à la chambre de mesure (Me) par un cinquième capillaire (K5).

5. Dispositif selon l'une des revendications 2 à 4, caractérisé en ce que le deuxième capillaire (K2) part d'une zone de la deuxième chambre d'alimentation (A2) qui se situe sensiblement à mi-chemin entre son orifice de remplissage (F2) et le fond (B) opposé à l'orifice de remplissage.

6. Dispositif selon l'une des revendications 2 à 5, caractérisé en ce que le cinquième capillaire (K5) part d'une zone (A2a) de la deuxième chambre d'alimentation (A2)qui se situe sensiblement au niveau de son orifice de remplissage (F2).

7. Dispositif selon l'une des revendications précédentes, caractérisé en ce que le réseau de capillaires (KA) forme une entrée de la chambre à résidus (Abf).

8. Dispositif selon l'une des revendications précédentes, caractérisé en ce qu'au moins un des capillaires (K1, K2, K4, K5) conduisant à une chambre contenant un support capillaire (T2, T3, T1) est relié à la chambre (V1, V2, Me) par l'intermédiaire d'une valve unidirectionnelle à écoulement capillaire statique n'autorisant le passage que dans la direction de cette chambre (V1, V2, Me).

9. Dispositif selon l'une des revendications précédentes, caractérisé en ce que la chambre à résidus (Abf) et/ou la chambre de mélange (Mi) sont chacune munies d'au moins un évent (L1, L2).

10. Dispositif selon l'une des revendications précédentes, caractérisé en ce que la deuxième chambre intermédiaire (V2) est montée en série avec une troisième chambre intermédiaire (V3) dans laquelle au moins un réactif auxiliaire se trouve placé sur un quatrième support à effet capillaire (T4).

11. Dispositif selon l'une des revendications précédentes, caractérisé en ce que la deuxième chambre d'alimentation (A2) est reliée à la première chambre intermédiaire (VI) par l'intermédiaire d'une quatrième chambre intermédiaire (V4) dans laquelle se trouve un support à effet capillaire (T5).

12. Dispositif selon l'une des revendications précédentes, caractérisé en ce que la deuxième chambre d'alimentation (A2) est subdivisée par une cloison (T) en deux zones (A2a, A2b) dont l'une, la zone (A2a) est reliée au deuxième capillaire (K2) et au deuxième orifice de remplissage (F2) et l'autre, la zone (A2b), au cinquième capillaire (K5).

13. Dispositif selon l'une des revendications précédentes, caractérisé en ce que les capillaires (K1, K2, K3, K4, K5) présentent un diamètre hydraulique de 0,05 à 2 mm.

14. Dispositif selon la revendication I3, caractérisé en ce que les capillaires (K1, K2, K3, K4, K5) présentent un diamètre hydraulique de 0,2 à 0,6 mm.

15. Dispositif selon l'une des revendications précédentes, caractérisé en ce que les supports à effet capillaire (T1, T2, T3, T4, T5) sont constitués par du papier, du non tissé, du tissu, du tricot ou du matériau de membrane poreux.

16. Dispositif selon l'une des revendications précédentes, caractérisé en ce que les chambres (A1, A2, Me, Mi, Abf, V1, V2, V3, V4), les capillaires (K1, K2, K3, K4, K5), le réseau de capillaires (KA) et les valves unidirectionnelles à écoulement capillaire (E1, E2, E3, E4) sont incorporés dans une surface d'une première plaque (P1) qui est recouverte d'une deuxième plaque (P2), l'une au moins des plaques (P1, P2) étant transparente au moins dans la zone de la chambre de mesure (Me).

17. Dispositif selon la revendication 16, caractérisé en ce que les plaques (P1, P2) sont constituées par du verre ou d'une matière synthétique.

18. Dispositif selon la revendication 17, caractérisé en ce que les plaques (P1, P2) sont constituées par du polyacrylate ou du polystyrène.

19. Valve unidirectionnelle à écoulement capillaire statique, destinée surtout à un dispositif selon l'une des revendications précédentes, caractérisée par une chambre (2) dans laquelle débouchent un capillaire d'amenée (4) et un capillaire d'écoulement (6), les surfaces de paroi (8) du capillaire d'amenée (4) formant avec les surfaces de parois contiguës (10, 12, 14, 16) de la chambre (2) des angles (α) qui, par leur valeur moyenne, sont supérieurs à celle des angles (β) qui comprennent les surfaces de paroi (18) du capillaire d'écoulement (6) avec les surfaces de paroi contiguës (20, 22, 24) de la chambre (2).

20. Valve selon la revendication 19, caractérisée en ce que la chambre (2) comporte des faces latérales (14,16) divergeant à angle aigu dans la zone du sommet desquelles débouche le capillaire d'amenée (4) et une face latérale (20, 22, 24) se trouvant à l'opposé de la zone du sommet dans la zone médiane de laquelle le capillaire d'écoulement (6) débouche à angle droit.

21. Valve selon la revendication 19 ou 20, caractérisée en ce que la chambre (2) comporte un fond (12) dans lequel débouche un canal d'aération (26).

# FIG . 1

# FIG.2

FIG . 3

## FIG. 4

## FIG. 5